# FASCICULE DE BREVET EUROPEEN

(11) **EP 0 814 764 B1**
(45) Date de publication et mention de la délivrance du brevet: **12.05.1999**
(21) Numéro de dépôt: 96941737.7
(22) Date de dépôt: 10.12.1996
(51) Int. Cl.: A61K 7/48, A61K 7/06

(54) **COMPOSITIONS COSMETIQUES A BASE DE POLYCONDENSATS IONISABLES MULTISEQUENCES POLYSILOXANE/POLYURETHANE ET/OU POLYUREE EN SOLUTION ET UTILISATIONS**
KOSMETISCHE ZUSAMMENSETZUNGEN AUF BASIS VON IONISIERBAREN MULTIBLOCK POLYKONDENSATEN AUS POLYSILOXAN/POLYURETHAN UND/ODER POLYHARNSTOFF IN LÖSUNG UND VERWENDUNG
COSMETIC COMPOSITIONS CONTAINING MULTI-BLOCK IONISABLE POLYSILOXANE/POLYURETHANE AND/OR POLYUREA POLYCONDENSATES IN SOLUTION, AND USES THEREOF

(30) Priorité: 05.01.1996 FR 9600098
(43) Date de publication de la demande: 07.01.1998
(73) Titulaire: L'OREAL, 75008 Paris (FR)
(72) Inventeur: MOUGIN, Nathalie, F-75011 Paris (FR); MONDET, Jean, F-93600 Aulnay-sous-Bois (FR)
(74) Mandataire: Dodin, Catherine
(86) Numéro de dépôt international: FR9601976
(87) Numéro de publication internationale: WO9725021

(56) Documents cités:
- EP-A- 0 636 361

## Description

La présente invention a pour objet de nouvelles compositions cosmétiques ou dermatologiques à propriétés filmogènes contenant des solutions de polycondensats ionisables multiséquencés particuliers, ainsi que diverses de leurs utilisations possibles, notamment dans le domaine des traitements cosmétiques (i.e. par voie topique) de la peau, des cheveux, des ongles et autres matières kératiniques.

Il est d'usage courant de mettre en oeuvre dans des formulations cosmétiques, en particulier dans des produits capillaires (shampooings, après-shampooings, lotions ou gels coiffants ou traitants, laques ou lotions de mise en forme, de mise en plis ou de fixation, etc...) ou dans des produits de maquillage (tels que par exemple vernis à ongle, mascaras, eye-liners, et autres), une proportion variable, selon la nature et la destination de la formulation, d'au moins une substance filmogène permettant de, ou visant à, conférer au support sur lequel elle est appliquée (c'est-à-dire, ici, l'une des parties superficielles du corps, telle que cheveux, cils, poils, peau, ongles,...) certaines caractéristiques améliorées. Ainsi, par exemple, dans le cas particulier du traitement d'une chevelure, on recherche avant tout par cette technique plus de tenue et plus de douceur pour les cheveux, alors que dans le cas plus particulier des ongles, on recherche principalement l'obtention d'un film protecteur brillant et dur, adhérant parfaitement sur ces derniers.

Une résine filmogène, pour être satisfaisante dans des applications cosmétiques, se doit de présenter certaines caractéristiques ou propriétés contraignantes, parmi lesquelles on peut plus particulièrement citer, et ceci de manière non limitative, d'abord une très bonne affinité/compatibilité/innocuité vis-à-vis des différentes matières kératiniques (peau, cheveux et autres), ensuite de bonnes propriétés filmogènes par rapport à ces dernières (qualité et régularité du film déposé), et enfin de bonnes propriétés de rémanence (adhésivité, solidité), c'est-à-dire qu'elle doit être difficilement éliminable de son support par de simples lavages à l'eau ou à l'aide de détergents (shampooings) par exemple. Dans le cas des vernis à ongles, le film doit par ailleurs posséder une bonne résistance à l'abrasion mécanique. D'une manière générale, on notera qu'il est souvent difficile, dans la pratique, de trouver une substance filmogène susceptible de convenir effectivement à plusieurs, ou à toutes, les différentes applications cosmétiquement envisagables pour cette dernière (problème du compromis acceptable).

A certains égards, les substances filmogènes connues à ce jour, et en particulier celles mentionnées ci-avant, conviennent mal pour l'obtention de compositions présentant de bonnes propriétés cosmétiques, en raison notamment d'un manque notable de rémanence, en particulier de résistance à l'eau.

Un autre problème réside dans le fait que les films ainsi obtenus, en particulier dans le cadre des applications de type capillaires ou mascaras, présentent une brillance insuffisante. Cette brillance n'est en outre que faiblement rémanente, c'est-à-dire qu'elle disparaît rapidement sous l'action d'agents extérieurs (forte sensibilité à l'eau notamment).

Or, la brillance, ainsi que la rémanence de cette brillance, constitue aujourd'hui une propriété particulièrement recherchée dans le domaine de la cosmétique.

On voit donc qu'il existe actuellement dans l'état de l'art un fort besoin quant à pouvoir disposer de compositions filmogènes cumulant, et ceci pour un domaine varié d'applications possibles (cheveux, cils, peau, ongles,...), tous les avantages généralement recherchés ou désirables en cosmétique, à savoir notamment l'innocuité vis à vis des matières kératiniques, la facilité d'application et de mise en oeuvre, l'obtention de dépôts protecteurs fins et réguliers, la rémanence des propriétés adhésives, l'apport et la rémanence des propriétés de brillance, l'apport de douceur et de lubrification, de rigidité et de résistance à l'abrasion.

Pour répondre à ce besoin, on a proposé d'utiliser des mélanges de polymères permettant de combiner toutes ces propriétés, notamment des mélanges de polyorganosiloxanes (silicones) avec des polymères non-siliconés. En effet, on sait que les silicones apportent d'excellentes propriétés de surface conduisant à une bonne lubrification, une bonne brillance, une douceur au toucher sans apport de gras. Ces polymères ne présentent pas de bonnes propriétés mécaniques pour assurer une bonne filmification ; il faut donc leur associer d'autres polymères apportant des propriétés mécaniques. Les polyorganopolysiloxanes en particulier les polydiméthylsiloxanes sont incompatibles avec la plupart des polymères non-siliconés apportant des propriétés mécaniques.

Pour résoudre ces inconvénients, la demande de brevet français N° 2 708 199 enseigne la possibilité d'utiliser une suspension aqueuse stable constituée de fines particules solides, généralement sphériques, de polycondensat ionique multiséquencé Polysiloxane/Polyuréthane et/ou Polyurée, ces particules ayant été obtenues par mise en dispersion, dans une phase aqueuse appropriée, dudit polycondensat à l'état déjà synthétisé. Ce type de dispersion de polymère insoluble dans l'eau est appelée 〈〈pseudo-latex〉〉. Ces pseudo-latex présentent cependant certains inconvénients.

Ces polycondensats ionisables en suspension aqueuse ne permettent pas de potentialiser la conjugaison souhaitée des propriétés apportées d'une part par les segments silicones et d'autre part par les segments Polyuréthanes et/ou Polyurée. Cette additivité des propriétés ne peut être optimisée que s'il se produit pendant le séchage, une bonne séparation de phase entre les segments siliconés et les segments polyuréthanes et/ou polyurées de telle sorte qu'il y ait d'une part, à l'état solide, une véritable stratification des segments silicones en surface de la matrice du dépôt constituée par les segments polyuréthanes et/ou polyurée -c'est en effet à l'interface de cette matrice avec l'air que vont se manifester les propriétés spécifiques des silicones- et d'autre part de telle sorte que les segments silicones soient rassemblés à l'intérieur de la matrice de dépôt sous forme de phase dispersée.

Il est très difficile d'obtenir à partir des pseudo-latex constitués de polycondensats ionisables Polysiloxane/Polyuréthane et/ou Polyurée, une bonne séparation de phase conduisant à cette stratification des segments silicones en surface du dépôt et à la formation d'une phase siliconée à l'intérieur du dépôt et plus particulièrement lorsque les longueurs des séquences polysiloxanes et/ou polyuréthanes et/ou polyurées sont courtes. Il en résulte que l'additivité des propriétés telles que définies ci-dessus obtenue par ces pseudo-latex reste encore insuffisante. Ainsi, à la suite d'importantes recherches menées sur la question, il a été trouvé par la

Demanderesse, et ceci de façon inattendue et surprenante qu'il était possible d'améliorer de façon substantielle cette séparation de phase et par conséquent de potentialiser l'additivité des propriétés de surface apportées par les silicones et des propriétés mécaniques et/ou d'adhésion apportées par les polyuréthanes et/ou polyurées, en utilisant des solutions ou des émulsions de ces mêmes polycondensats ionisables multiséquencés Polysiloxane/Polyuréthane et/ou Polyurée dissous dans un système solvant aqueux, organique ou hydroorganique.

De plus, les polycondensats en solution de l'invention présentent par rapport aux pseudo-latex constitués par les mêmes polycondensats une meilleure rémanence du dépôt à l'action de l'eau ou des solutions de tensio-actifs (shampooings).

Conformément à la présente invention, il est donc maintenant proposé de nouvelles compositions cosmétiques qui sont caractérisées par le fait qu'elles comprennent, dans un support cosmétiquement acceptable, au moins une solution ou une émulsion d'au moins un polycondensat multiséquencé dont la chaîne est constituée par la répétition d'au moins une séquence polysiloxane et d'au moins une séquence polyuréthane et/ou polyurée ; ladite séquence polyuréthane et/ou polyurée comportant en outre des groupements ionisables et ledit polycondensat étant dissous dans un système solvant aqueux, organique ou hydroorganique.

On entend par 〈〈groupement ionisable〉〉,tout groupement susceptible d'être ionisé par une réaction de neutralisation d'une fonction acide ou basique portée par ledit groupement ou de quaternisation d'une fonction amine tertiaire portée par ledit groupement et de former ainsi un groupement anionique, cationique, amphotère ou zwitterionique.

Les polycondensats de l'invention sont essentiellement préparés selon un procédé en deux étapes. La première étape consiste en une réaction classique de polycondensation entre (i) un polymère polysiloxane (ou silicone) présentant une fonction hydroxy ou une fonction amine aux extrémités de sa chaîne (i.e un α,ω-dihydroxypolysiloxane, ou un α,ω-diaminopolysiloxane, ou un α,ω-aminohydroxy- ou hydroxyamino-polysiloxane), et (ii) un diisocyanate (présent en quantité stoechiométrique, ou en excès stoëchiométrique, c'est-à-dire à plus de 2 moles par mole de silicone) ce par quoi l'on obtient une nouvelle silicone présentant cette fois une fonction isocyanate à chacune de ses extrémités de chaîne; puis, dans une deuxième étape, on couple les chaînes du polycondensat obtenu précédemment au moyen d'un agent coupleur (en quantité variable choisie en fonction de la longueur de chaîne finale désirée) choisi parmi les diols et/ou les diamines et/ou les alcoolamines, de manière à obtenir finalement un nouveau polycondensat de plus longue chaîne.

Les réactions mises en oeuvre dans la première étape conduisent ainsi à un polysiloxane présentant à ses extrémités de chaîne, outre les fonctions isocyanates mentionnées ci-dessus, des motifs uréthane et/ou urée, et ceci selon les mécanismes classiques d'une réaction de condensation opérée entre (i) une fonction isocyanate, telle que portée par le diisocyanate de départ, et (ii) une fonction alcool (création dans ce cas d'un motif uréthane) ou d'une fonction amine (formation dans ce cas d'une fonction urée), telles que portées par le polysiloxane de départ, à savoir :

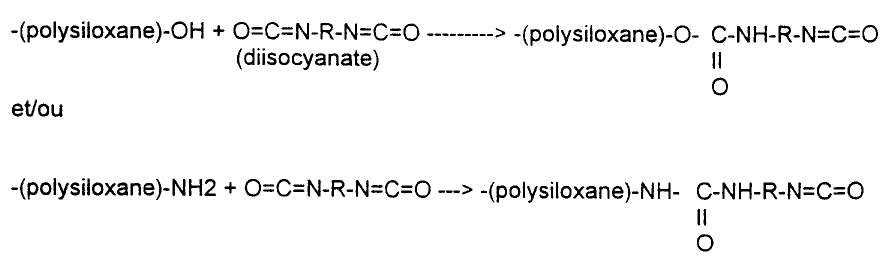

Les polycondensats obtenus à l'issue de cette première étape peuvent donc être en fait définis par la formule générale (1) suivante : 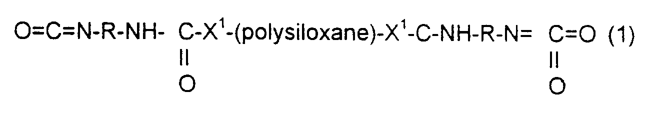 dans laquelle X¹ peut donc représenter, séparément ou conjointement, -O- ou -NH-.

Dans la deuxième étape, les fonctions alcools et/ou amines de l'agent coupleur (agent coupleur que l'on peut ici symboliser de manière commode par OH-B-OH, ou NH2-B-NH2 ou bien encore NH2-B-OH) viennent alors réagir, et ceci selon les mêmes mécanismes que ceux exposés pour la première étape, soit avec les fonctions isocyanates portées en bout de chaîne par le polycondensat polysiloxane de formule (1) ci-dessus, soit avec des fonctions isocyanates portées par du diisocyanate libre, lorsque ce dernier a été introduit en excès stoechiométrique lors de la première étape, donnant ainsi naissance dans la chaîne (plus longue) du nouveau polycondensat obtenu à une sucession de motifs uréthane et/ou urée, c'est-à-dire à des séquences de type polyuréthane et/ou polyurée symbolisables par la formule (2) : 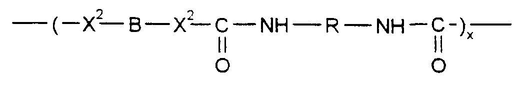 dans laquelle X² représente -O- ou -NH-, et x est une valeur correspondant substantiellement au nombre de moles d'agent coupleur mises en oeuvre dans la réaction.

Comme indiqué précédemment, on obtient donc ainsi finalement un polycondensat constitué par la répétition de séquences polysiloxanes (correspondant simplement au polysiloxane d'origine, et tel qu'il apparait dans la formule (1)) et de séquences polyuréthanes et/ou polyurées (formule (2)).

Selon un aspect extrêmement important de l'invention, les agents coupleurs (c'est-à-dire en fait le radical B) portent des groupements ionisables, c'est-à-dire des groupements qui, et respectivement, lorsqu'ils sont soumis à l'action d'une base, donnent des groupement anioniques (c'est le cas par exemple des groupements carboxyliques) et lorsqu'ils sont soumis à l'action d'un acide ou d'une quaternisation, donnent des groupements cationiques (cas par exemple d'une amine tertiaire). La neutralisation des groupements anionisables (respectivement cationisables) par la base (respectivement par l'acide) peut être effectuée de façon partielle ou totalement, selon les quantité d'agents neutralisants mises en oeuvre.

Mais d'autres caractéristiques, aspects et avantages de l'invention apparaitront maintenant de manière plus claire à la lecture de la description détaillée et complète qui va suivre, ainsi que des divers exemples concrets, mais nullement limitatifs, destinés à l'illuster.

Comme indiqué précédemment, la chaîne du polycondensat ionisable utilisé dans le cadre de la présente invention est essentiellement caractérisée par le fait qu'elle est constituée par la répétition (ou alternance) d'au moins une séquence de type polysiloxane et d'au moins une séquence de type polyuréthane et/ou polyurée, lesdites séquences polyuréthanes et/ou polyurées comportant des groupement ionisables. D'autres séquences de nature chimique différente peuvent se pprésenter dans la chaîne du polycondensat. On peut citer par exemple des séquences Polyéthers telles que Polyoxyéthylène, Polyoxypropylène, Polytétraméthylène oxyde et/ou des séquences Polyesters telles que Polyadipate d'éthylèneglycol, Polysébacate de néopentylglycol, Polytéréphtalate d'éthylèneglycol.

La répétition des séquences ci-dessus peut être de type aléatoire, mais elle est de préférence de type régulièrement alternée. En outre, le rapport en nombre entre les séquences de type polyuréthane et/ou polyurée et les séquences de type polysiloxane est généralement compris entre 1 et 10, de préférence entre 1 et 3.

Les poids moléculaires des polycondensats Polysiloxanes-Polyuréthane/Polyurée peuvent varier dans de larges limites, en particulier entre 2000 et 500 000, mais de préférence entre 3000 et 250 000.

De préférence, la séquence polysiloxanique répond à la formule générale (I) suivante : 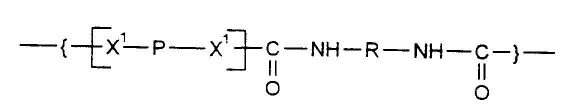 dans laquelle :
- P est un segment polysiloxane,
- X¹ représente, séparement ou conjointement, -O- ou -NH-,
- et R (qui n'est autre que le motif du diisocyanate tel que mentionné ci-avant) est un radical divalent choisi parmi les radicaux alkylènes de type aromatique, aliphatique ou cycloaliphatique.

De préférence, le segment polysiloxane P répond à la formule générale (I') suivante : 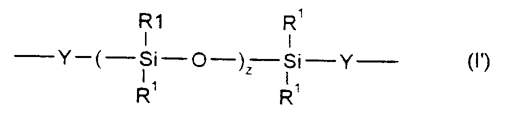 dans laquelle les radicaux R¹, qui peuvent être identiques ou différents, sont choisis parmi d'une part les radicaux hydrocarbonés monovalents en C₁-C₂₀ exempts ou substantiellement exempts d'insaturations éthyléniques et, d'autre part, les radicaux aromatiques, Y représente un radical hydrocarboné bivalent, et z un nombre entier tel que le poids moléculaire moyen du segment polysiloxane soit compris entre 300 et 10 000.

De préférence, Y est un radical bivalent choisi parmi les radicaux alkylènes de formule -(CH₂)ₐ-, dans laquelle a représente un nombre entier pouvant être compris entre 1 et 10.

A titre de radicaux R¹ convenant dans le cadre de l'invention, on peut plus particulièrement citer les radicaux alkyles, et notamment les radicaux méthyle, éthyle, propyle, isopropyle, butyle, pentyle, hexyle, octyle, décyle, dodécyle et octadécyle, les radicaux cycloalkyles, en particulier le radical cyclohexyle, les radicaux aryles, notamment phényle et naphtyle, les radicaux arylalkyles, notamment benzyle et phényléthyle, ainsi que les radicaux tolyle et xylyle. On notera que, selon l'invention, il est important que le segment polysiloxane soit exempt, ou substantiellement exempt, de motifs du type Si-H ou Si-R¹ dans lequel R¹ représenterait un radical hydrocarboné présentant des insaturations éthyléniques, et ceci de manière à éviter toute réticulation intempestive du polycondensat sur lui-même.

Selon un mode particulièrement préféré de réalisation de la présente invention, le segment polysiloxane P présent dans les polycondensats répond à la formule (I'') suivante: 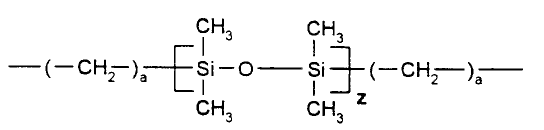 dans laquelle a et Z sont des valeurs telles que définies ci-avant.

Concernant maintenant les séquences polyuréthanes et/ou polyurées rentrant dans la constitution des polycondensats qui sont utilisés dans le cadre de l'invention, celles-ci répondent de préférence à la formule générale (II) suivante : 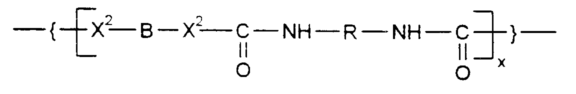 dans laquelle :
- X² représente, séparément ou conjointement, -O- ou -NH-,
- R (qui, comme ci-avant pour la formule (I), n'est autre que le motif du diisocyanate utilisé pour conduire la réaction de condensation) est tel que défini ci-dessus pour les séquences de formule (I),
- x (qui, comme indiqué précédemment dans la description correspond substantiellement au nombre de moles d'agents coupleurs utilisées dans le procédé de synthèse du polycondensat) est un nombre entier pouvant varier de 1 à 10, et de préférence de 1 à 3,
- et B (qui n'est autre que le motif apporté par l'agent coupleur tel que mentionné ci-avant) est un radical hydrocarboné bivalent porteur d'une charge ionique, positive ou négative.

A titre de radicaux B porteurs de groupements anioniques (i.e de charges négatives), on peut plus particulièrement citer ceux qui sont porteurs de groupement présentant une ou des fonction(s) carboxylique(s) et/ou une ou des fonctions sulfoniques, lesdites fonctions carboxyliques et/ou sulfoniques étant sous forme libre ou bien neutralisées partiellement ou totalement par une base minérale ou organique, comme cela sera expliqué plus en détails par la suite.

Ainsi, parmi les radicaux B bivalents porteurs de fonctions carboxyliques ou sulfoniques convenant particulièrement bien dans le cadre de la présente invention, on peut mentionner ceux de formule (III): 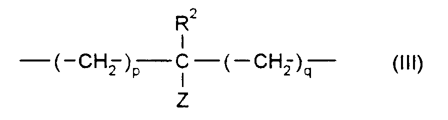 dans laquelle R² représente un radical alkyle, linéaire ou ramifié, en C₁-C₃, Z une fonction acide carboxylique (-COOH) ou acide sulfonique (-SO₃H) ou un sel desdites fonctions acides (fonctions carboxylate et sulfonate, respectivement), et p et q, qui peuvent être identiques ou différents, des nombres entiers compris entre 1 et 5,
et ceux de formule (III') : 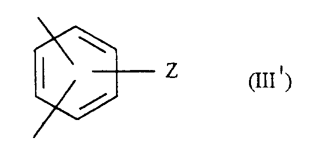 dans laquelle Z a la signification ci-dessus,

A titre de radicaux B porteurs de groupements cationiques (i.e. de charges positives), on peut plus particulièrement citer ceux qui sont porteurs de groupements de type amines tertiaires, lesdites amines tertiaires étant soit non-neutralisées, soit pour partie ou totalement neutralisées (présence de motifs -NH⁺-) soit quaternisées, comme cela sera expliqué plus en détails par la suite.

Ainsi, parmi les radicaux B bivalents porteurs de fonctions amines tertiaires cationisables convenant particulièrement bien dans le cadre de la présente invention, on peut mentionner ceux de formule : 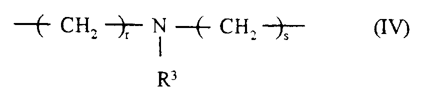 dans laquelle R³ représente un radical alkyle, linéaire ou ramifié, en C₁-C₄, et r et s deux nombres entiers, identiques ou différents, qui peuvent être compris entre 1 et 10.
Sous forme neutralisée ou quaternisée, les radicaux B ci-dessus deviennent alors : 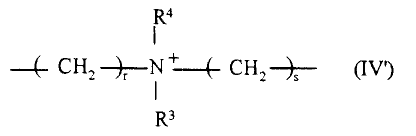 formule dans laquelle R³ a la signification ci-dessus, et R⁴ représente soit l'hydrogène (neutralisation) soit un radical alkyle, linéaire ou ramifié, en C₁-C₁₀ ou un cycle aromatique (quaternisation).

Selon l'invention, les taux de neutralisation des fonctions anionisables ou cationisables peuvent être compris entre 0 et 100%, de préférence entre 10 et 100%.

Les radicaux B du coupleur porteur d'une charge ionique peuvent être utilisés seuls ou en mélange avec d'autres radicaux B' provenant de coupleurs ne portant pas de groupe ionisable.

Concernant enfin les radicaux R plus particulièrement préférés selon la présente invention et rentrant dans le cadre de la définition des séquences de formules (I) et (II) données ci-avant, on peut mentionner ceux de formules : 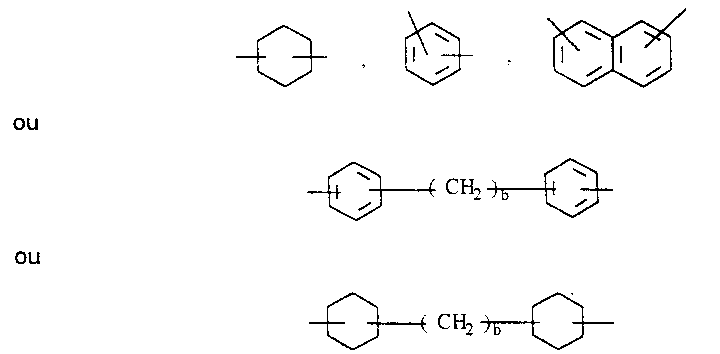 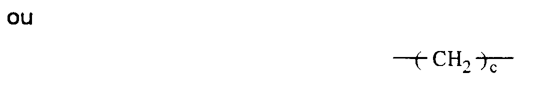 dans lesquelles b est un nombre entier compris entre 0 et 3 , et c un nombre entier compris entre 1 et 20, de préférence compris entre 2 et 12.

Parmi les radicaux bivalents R particulièrement préférés rentrant dans le cadre des formules ci-dessus, on peut citer les radicaux hexaméthylène, 4,4'-biphénylèneméthane, 2,4- et/ou 2,6-tolylène, 1,5-naphtylène, p-phénylène, méthylène 4,4-biscyclohéxyle et le radical divalent dérivé de l'isophorone.

Le procédé de synthèse des polycondensats utilisés dans le cadre de la présente invention va maintenant être développé un peu plus en détails. Dans ces grandes lignes, ce procédé correspond à celui déja indiqué en début de description.

On fait réagir, dans un solvant organique, un α,ω-dihydroxy et/ou diamino et/ou aminohydroxy et/ou hydroxyaminopolysiloxane répondant à la formule générale suivante:

X³-P-X³

dans laquelle P a la signification donnée ci-avant (segment polysiloxane), et X³ représente, conjointement ou séparément, -OH ou -NH₂,
avec un excès stoechiométrique d'un diisocyanate de formule :

O=C=N-R-N=C=O

dans laquelle R a la signification donnée ci-avant,
puis à coupler les chaînes du polycondensat obtenu précédemment par au moins un diol et/ou une diamine et/ou une alcool amine répondant à la formule :

X⁴-B-X⁴

dans laquelle B a la signification donnée ci-avant, et X⁴ représente -OH ou -NH₂, à une température comprise entre 40 et 100°C, en présence d'un sel d'étain en tant que catalyseur. Ce diol et/ou cette diamine X⁴-B-X⁴ peuvent être utilisés seuls ou en mélange avec un ou plusieurs diols ou diamines ou aminoalccols ne comportant pas de groupes ioisables par exemple 1,4-butanediol.
Le solvant organique utilisé à ces étapes est de préférence choisi dans le groupe constitué par l'acétone, la méthyléthylcétone, le tétrahydrofuranne et le 1,2-dichloroéthane, ces solvants étant inertes vis-à-vis des groupes isocyanates.

Le sel d'étain est, quant à lui, de préférence choisi parmi l'éthyl-2 hexanoate d'étain et le dibutyl dilaurate d'étain.

Dans le cadre de la mise en oeuvre du procédé ci-dessus, les diisocyanates particulièrement préférés sont choisis, seuls ou en mélanges, parmi le diphénylméthane 4,4'-diisocyanate et le méthylène 4,4'-bis-dicyclohexyldiisocyanate, et les agents coupleurs particulièrement préférés sont choisis, seuls ou en mélanges, parmi l'acide diméthylol propionique, la N-méthyldiéthanolamine, le 1,3-diaminopropane et l'éthanolamine, étant bien entendu que la possibilité de mélange coupleur acide/coupleur amine est exclue.

Le polycondensat polysiloxane-Polyuréthane/Polyurée ainsi obtenu peut être ensuite éventuellement purifié, par exemple par précipitation dans un solvant non polaire tel que le cyclohexane.

Conformément à l'invention, ce polycondensat, éventuellement purifié, est ensuite soit utilisé tel quel, soit neutralisé à l'aide d'un agent neutralisant convenable qui peut être soit une base minérale ou organique lorsque le radical B tel que ci-avant défini est porteur de fonctions anionisables telles que par exemple des fonctions acides carboxyliques et/ou sulfoniques, soit un acide minéral ou organique lorsque ledit radical B est porteur de fonctions cationisables telles que par exemple des fonctions amines tertiaires ; soit quaternisé à l'aide d'un halogénure d'alkyle, d'un sel d'acide portant un halogène mobile ou une sultone ( par exemple propane sultone) lorsque ledit radical B est porteur de fonctions amines tertiaires.

Selon l'invention, le taux de neutralisation peut aller de 0% à 100%, de préférence de 10 à 100 %.

Il va de soi que la nature de l'agent neutralisant qu'il conviendra d'utiliser pour neutraliser le polycondensat Polysiloxane-Polyuréthane/Polyurée sera fonction de la nature des fonctions ionisables portées par ce dernier.

Lorsque ledit polycondensat comporte une fonction anionisable telle que par exemple une fonction acide carboxylique ou sulfonique, l'agent neutralisant peut être une base minérale telle que la soude, la potasse ou l'ammoniaque, ou une base organique telle qu'un aminoalcool choisi notamment parmi le 2-amino 2-méthyl 1-propanol (AMP), la triéthanolamine, la triisopropanolamine (TIPA), la monoéthanolamine, la diéthanolamine, la tri[(2-hydroxy) 1-propyl]amine, le 2-amino 2-méthyl 1,3-propanediol (AMPD), et le 2-amino 2-hydroxyméthyl 1,3-propanediol ou bien une diamine telle que la lysine.

Lorsque le polycondensat comporte une fonction cationisable du type amine tertiaire, l'agent neutralisant peut être un acide minéral tel que l'acide chlorhydrique, ou un acide organique tel que l'acide lactique, l'acide glycolique ou l'acide mandélique. L'agent neutralisant peut être aussi un agent quaternisant de la fonction amine tertiaire, comme par exemple les halogénures d'alkyles et en particulier le iodure de méthyle ou le bromure d'éthyle. Il peut être également un sel d'acide portant un halogène mobile ou un ester cyclique d'acide sulfonique.

On réalise ensuite une solution ou une émulsion solvant organique-dans-eau du polycondensat conforme à l'invention en l'incorporant dans un système solvant organique, aqueux ou hydro-organique.

Les solvants organiques des polycondensats de l'invention utilisés selon l'invention sont choisis de préférence parmi l'acétone, la méthyléthylcétone, l'acétate de méthyle, l'acétate de butyle, l'acétate d'éthyle, l'isopropanol, l'éthanol, le diméthoxyéthane, le diéthoxyéthane, un ester d'éthylèneglycol ou de propylèneglycol, un ether d'éthylèneglycol ou de propylèneglycol, ou un ether ester d'éthylèneglycol ou de propylèneglycol et leurs mélanges.

Suivant le type d'application choisi, on peut utiliser, parmi ces solvants des polycondensats de l'invention, un solvant ou un mélange de solvants miscibles à l'eau dont l'un (servant de diluant) s'évapore avant l'eau de façon à permettre la dissolution du polymère dans un solvant pendant toute la durée du séchage de la formulation appliquée sur la matière kératinique traitée. Parmi les solvants miscibles à l'eau, on peut citer diméthoxyéthane et un mélange de diméthoxyéthane/diéthoxyéthane. Si la formulation envisagée nécessite pour l'application donnée la présence d'eau, la solution organique du polymère peut dans ce cas être diluée dans l'eau pour former une émulsion solvant organique-dans-eau. Cette émulsion pourra être autostabilisée par les charges ioniques portées par le polycondensat qui se placent d'elles-mêmes à l'interface avec l'eau ou bien stabilisée si nécessaire par des agents stablisants tels que des tensio-actifs ou des agents gélifiants présents dans la phase aqueuse

On peut également choisir un solvant ou un mélanges de solvants des polycondensats de l'invention non-miscibles à l'eau tels que le diéthoxyéthane. Si la formulation envisagée nécessite pour l'application donnée la présence d'eau, la solution organique du polymère peut dans ce cas être dispersée dans l'eau pour former une émulsion solvant organique-dans-eau. Cette émulsion pourra être autostabilisée par les charges ioniques portées par le polycondensat qui se placent d'elles-mêmes à l'interface avec l'eau ou stabilisée si nécessaire par des agents stablisants tels que des tensio-actifs ou des agents gélifiants présents dans la phase aqueuse. Dans ce cas, le solvant ou l'un des solvants organiques utilisés pour dissoudre le polycondensat présente de préférence un point d'ébullition supérieur à celui de l'eau. On peut utiliser notamment le diéthoxyéthane.

Une forme particulièrement préférée de système solvant des polycondensats de l'invention consiste à utiliser un mélange de solvants de polarités différentes comprenant au moins un solvant (A) dit 〈〈global〉〉 du polycondensat (pour les séquences polysiloxanes et les séquences polyuréthanes et/ou polyurées) et au moins un solvant (B) moins polaire que (A) qui va solvater plus spécifiquement les séquences polysiloxanes. Pour favoriser la stratification des séquences polysiloxanes sur la matrice de dépôt, on choisira de préférence un solvant (B) dont la vitesse d'évaporation est plus lente que (A).

Parmi les solvants globaux (A), on peut citer l'acétone, la méthyléthylcétone, l'acétone, la méthyléthylcétone, l'acétate de méthyle, l'acétate de butyle, l'acétate d'éthyle, l'isopropanol, l'éthanol, le diméthoxyéthane, le diéthoxyéthane, un ester d'éthylèneglycol ou de propylèneglycol, un ether d'éthylèneglycol ou de propylèneglycol, ou un ether ester d'éthylèneglycol ou de propylèneglycol et leurs mélanges.

Parmi les solvants (B), on peut citer les hydrocarbures en particulier les hydrocarbures ramifiés comme les isoparaffines, l'isododécane, les silicones cycliques du type D₄, D₅ ou D₆.

La neutralisation peut être réalisée *in situ* dans la solution du polycondensat Polysiloxane-Polyuréthane/Polyurée dans le système solvant par addition de la quantité déterminée d'agent neutralisant.

Comme indiqué précédemment, les compositions cosmétiques selon l'invention, qui contiennent donc, dans un support cosmétiquement acceptable, des polycondensats tels que ci-dessus définis, présentent, pour des applications aussi variées que celles rencontrées par exemple dans le domaine du capillaire, du maquillage ou bien encore des soins de la peau, ou de tout autre domaine cosmétique dans lequel l'utilisation d'une substance filmogène est désirable ou recherchée, des propriétés tout à fait remarquables, en particulier au niveau de leurs propriétés filmogènes et de brillance, de leur aptitude à conserver ces propriétés dans le temps face à l'action d'agents extérieurs (rémanence) et aussi de leur propriétés de douceur, de lubrification et de résistance à l'abrasion.

Parmi les applications préférentiellement visées par la présente invention, et les différents effets bénéfiques obtenus dans ces dernières, on peut plus particulièrement mentionner :
- le domaine des produits capillaires (lavage, soin ou beauté des cheveux), où les compositions selon l'invention, en particulier sous forme d'aérosols, de mousse, de shampooings, d'après-shampooings, de lotions ou de gels coiffants ou traitants, laques ou lotions de mise en forme ou de mise en plis ou encore de fixation, permettent d'apporter aux cheveux brillance, douceur, facilité de coiffage (phénomène d'"individualisation" des cheveux au moment du dépôt de la composition), meilleur toucher, ainsi que la rémanence (c'est-à-dire le maintien durable, même sous l'action d'agents extérieurs) de ces propriétés.
- le domaine des produits de maquillage, en particulier pour le maquillage des ongles et des cils, où les compositions selon l'invention, sous forme de vernis à ongle, de mascaras ou de eye-liners par exemple, permettent d'apporter, dans le cas du maquillage des cils, les mêmes avantages que ceux évoqués précédemment pour le traitement des cheveux, et, dans le cas des vernis à ongles (où les compositions peuvent être utilisées comme filmogène seule ou comme additif filmogène), brillance, meilleure mouillabilité de l'ongle, rémanence du film et de sa brillance aux lavages, meilleure résistance à l'abrasion (apport de glissant par lubrification des surfaces), meilleure rigidité.
- dans le domaine des produits de soin de la peau (crèmes, laits, lotions, masques, sérums, produits solaires), où les compositions selon l'invention permettent plus particulièrement d'apporter brillance, meilleure mouillabilité et résistance aux lavages à l'eau (produits solaires).

La proportion en polycondensat dans les compositions cosmétiques (hors vernis à ongles) est généralement comprise entre 0,5 et 50%, et de préférence entre 1 et 20% en poids par rapport au poids total de la composition. Dans le cas des vernis à ongles, cette proportion peut aller jusqu'à 30% en poids.

Les compositions peuvent en outre, et bien entendu, contenir divers adjuvants destinés à la rendre acceptable dans une application cosmétique particulière.

Les compositions selon l'invention peuvent contenir des filtres solaires UV-A ou UV-B ou à bande large et être utilisées ainsi comme produits anti-solaires.

Les compositions selon l'invention peuvent par ailleurs contenir des additifs cosmétiques conventionnels choisis parmi les corps gras, des solvants organiques, des silicones, des agents épaississants, des adoucissants, des agents anti-mousse, des agents hydratants, des humectants, des agents traitants (agents anti-chute, anti-pélliculaire,...), des polymères anioniques, non ioniques ou amphotères ou leurs mélanges, des antiperspirants, des agents alcanisants, des colorants, des pigments, des parfums, des conservateurs et des agents propulseurs lorsque les compositions se présentent sous forme aérosol.

Plus précisémment, comme corps gras, on peut utiliser une huile ou une cire ou leurs mélanges, des acides gras, des alcools gras, des esters d'acides gras tels que les triglycérides d'acides gras en C₆-C₁₈, de la vaseline, de la paraffine, de la lanoline, de la lanoline hydrogénée ou acétylée.
Parmi les huiles, on peut citer les huiles minérales, animales, végétales ou les huiles de synthèse et notamment l'huile de vaseline, de paraffine, de ricin, de jojoba, de sésame, ainsi que les huiles et les gommes de silicones et les isoparaffines. Parmi les cires animales, fossiles, végétales, minérales ou de synthèse, on peut notamment citer la cire d'abeille, de caroube, de Candellila, l'ozokérite, les cires microcristallines ainsi que les cires et les résines de silicone.

Parmi les agents épaississants, on peut citer :
- les celuloses modifiées telles que l'hydroxyéthylcellulose, la méthylcellulose, l'hydroxypropylcellulose et la carboxyméthylcellulose. Parmi celles-ci, on peut citer notamment les gommes vendues sous la dénomination de "Cellosize QP 44001H par la Société Amercol,
- la gomme de caroube, la gomme de guar, la gomme de guar quaternisée vendue sous la dénomination de "Jaguar C-13-S" par la Société Meyhall la gomme d'hydroxypropylguar, la gomme de xanthane,
- les acides polyacryliques réticulés tels que les "Carbopol" de la Société Goodrich,
- les polymères poly(métha)crylates de glycéryle vendus sous les dénominations de "Hispagel" ou "Lubragel" par les Sociétés Hispano Quimica ou Guardian,
- la polyvinylpyrrolidone,
- l'alcool polyvinylique,
- les polymères et les copolymères réticulés d'acrylamide, tels que ceux vendus sous les dénominations de "PAS 5161" ou "Bozepol C" par la Société Hoechst, de "Sepigel 305" par la Société Seppic, ou de "Salcare SC95" par la Société Allied Colloïd, ou encore
- les homopolymères réticulés de chlorure de méthacryloyloxyéthyltriméthylammonium vendus sous la dénomination de "Salcare SC95" par la Société Allied Colloïd.

On va maintenant donner à titre d'illustration de l'invention plusieurs exemples de préparation de polycondensats Polysiloxane-Polyuréthane ainsi que de leurs solutions ou émulsions et de compositions cosmétiques les contenant.

Les synthèses conduisant aux polycondensats multiséquencés Polysiloxane-Polyuréthane ont été réalisées à partir de prépolymères du type polydiméthylsiloxane α,ω hydroxyorganofonctionnels (produits commerciaux vendus par la Société Goldschmidt sous les dénominations Tegomer H-Si 2111 et Tegomer H-Si 2311) de structure : 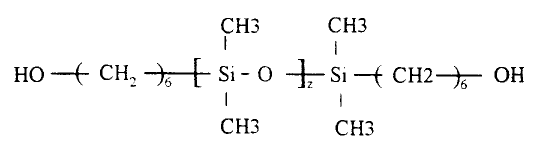 et ayant en outre les caractéristiques rassemblées dans le tableau ci-dessous :

| Nom commercial | Tegomer H-Si 2111 | Tegomer H-Si 2311 |
|---|---|---|
| Groupes fonctionnels | | |
| | groupes hydroxyles primaires | |
| Fonctionnalité | 2 | 2 |
| nombre de motifs z | environ 10 | environ 30 |
| Indice Hydroxyle (mg KOH/g) | 120 (+/-10) | 45 (+/-5) |
| viscosité à 25°C (cP) | 85 (+/-10) | 115 (+/- 15) |
| Poids moléculaire moyen en nombre (Mn) | 700 | 2 200 |

Dans la suite, ces deux produits commerciaux seront appelés par commodité SIL 700 et SIL 2200 (nomination basée sur leur poids moléculaire respectif).

### Exemple 1

Dans cet exemple, on a préparé un polycondensat Polysiloxane-Polyuréthane anionisable de structure théorique : 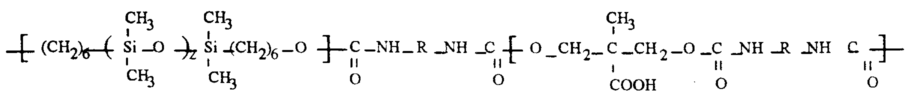 dans laquelle R représente : 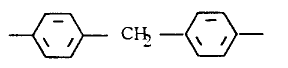 et correspondant à la réaction entre :
- 1 mole de SIL 700 (prépolymère polysiloxane)
- 2 moles de 4,4' diphénylméthanediisocyanate (ci-après dénommé MDI)
- et 1 mole d'acide diméthylolpropionique (agent coupleur, ci-après dénom mé DMPA),
ces valeurs étant rapportées à 1 mole de SIL 700.

Dans un réacteur cylindrique pourvu d'une agitation centrale du type ancre, d'un thermomètre, d'un réfrigérant, d'une arrivée de barbotage d'azote et surmonté d'une ampoule à introduction, on introduit, sous courant d'azote, 50 g de MDI et 50 g de tétrahydrofurane (THF). La dissolution du mélange se fait sous agitation et à température ambiante.

Parallèlement, on a dissout 70 g de SIL 700 dans 70 g de THF, et la solution ainsi obtenue est versée dans l'ampoule à introduction située au dessus du réacteur. On introduit alors, sous agitation et courant d'azote, cette solution de SIL 700 dans le réacteur contenant la solution de MDI, en maintenant la température du milieu de réaction à 50°C par chauffage extérieur.
La coulée de la solution de SIL 700 dure 1H30 et la température du milieu réactionnel est maintenue à 50°C pendant toute la durée de l'introduction.
A la fin de la coulée, la réaction conduit à la formation quantitative d'un prépolymère Polysiloxane à extrémités α,ω diisocyanate.

On introduit ensuite (temps de coulée : 30 mn) dans le réacteur contenant le prépolymère ci-dessus, et ceci toujours sous agitation, barbotage d'azote et maintien de la température à 50°C, une solution de DMPA obtenue en dissolvant 13,4 g de DMPA dans 400 g de THF. Au début de la coulée, on introduit en outre dans le milieu de réaction 0,15 g de dibutyldilaurate d'étain qui sert de catalyseur. On laisse ensuite réagir le tout pendant 10 h, sous agitation et à 50°C.
La fin de la réaction peut être controlée en vérifiant par analyse infra-rouge l'absence de bandes d'adsorption -N=C=O à 2270 cm⁻¹. Si besoin est, on peut alors rajouter de l'éthanol au milieu réactionnel pour terminer la réaction et consommer totalement les groupes -N=C=O encore disponibles, dans ce cas, on peut par exemple rajouter de l'ordre de 10 ml d'éthanol et laisser à nouveau réagir le tout pendant 4 H à 50°C.

En fin de réaction, on obtient une solution organique (THF) du polycondensat désiré, lequel est ensuite récupéré et purifié par précipitation de ladite solution dans 5 l d'un mélange équi-volume (50/50) éther de pétrole/éther éthylique. Le rendement de récupération est de 90% en poids après séchage.

L'indice d'acide du polycondensat obtenu est de 46 (théorique : 42).

Son poids moléculaire moyen en nombre est de 5000.

Le polymère obtenu est soluble dans des solvants tels que le diméthoxyéthane, l'éthanol, l'isododécane et l'acétate d'éthyle.

### Exemple 2

On procède ici à la préparation d'un polycondensat Polysiloxane-Polyuréthane anionisable de même structure théorique que celle de l'exemple 1, mais obtenu cette fois à partir du prépolymère polysiloxane SIL 2200

Le mode opératoire suivi est donc identique à celui de l'exemple 1, mais les quantités de réactifs mises en jeu sont cette fois les suivantes :
- 80 g de SIL 2200 dissous dans 80 g de THF
- 18,2 g de MDI dissous dans 20 g de THF
- 4,9 g de DMPA dissous dans 200 g de THF
- 0,1 g de dibutyldilaurate d'étain
de manière à observer là encore les proportions 1 mole de SIL 2200 : 2 moles de MDI : 1 mole de DMPA.

En outre, la récupération et la purification du polycondensat final désiré se fait cette fois plus simplement par précipitation de la solution organique le contenant dans 5 l d'eau permutée.

Le rendement de récupération est alors de 92% en poids.

L'indice d'acide du polycondensat obtenu est de 21,7 (théorique : 19,8).

Son poids moléculaire moyen en nombre est de 6300.

Le polymère obtenu est soluble dans des solvants tels que le diméthoxyéthane, l'éthanol, l'isododécane et l'acétate d'éthyle.

### Exemple 3

Dans cet exemple, on a préparé un polycondensat Polysiloxane-Polyuréthane cationisable de structure théorique : 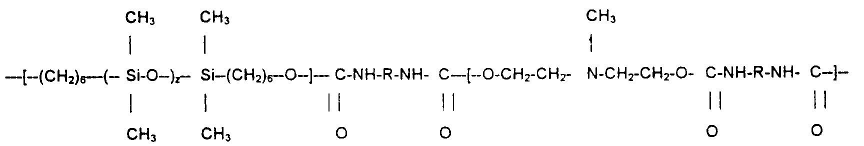 dans laquelle R représente : 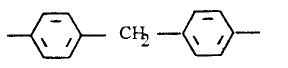 et correspondant à la réaction entre :
- 1 mole de SIL 700
- 2 moles de MDI
- 1 mole de N méthyldièthanolamine (agent coupleur, ci-après dénommé MEA) ces valeurs étant rapportées à 1 mole de SIL 700.

Dans le même réacteur que celui de l'exemple 1 et équipé de la même manière, on introduit, sous courant d'azote, 50 g de MDI et 50 g de THF. La dissolution du mélange se fait sous agitation et à température ambiante.
Parallèlement, on a dissout 70 g de SIL 700 dans 70 g de THF, et la solution ainsi obtenue est versée dans l'ampoule a introduction située au dessus du réacteur. On introduit alors, sous agitation et courant d'azote, cette solution de SIL 700 dans le réacteur contenant la solution de MDI, en maintenant la température du milieu de réaction à 50°C par chauffage extérieur.
La coulée de la solution de SIL 700 dure 1H30 et la température du milieu réactionnel est maintenue à 50°C pendant toute la durée de l'introduction.
A la fin de la coulée, on dilue le milieu de réaction par 350 g de THF, tout en maintenant la température à 50°C. La réaction a conduit à la formation quantitative d'un prépolymère Polysiloxane à extrémités α,ω diisocyanate.

On introduit ensuite (temps de coulée : 30 mn) dans le réacteur contenant le prépolymère ci-dessus, et ceci toujours sous agitation, barbotage d'azote et maintien de la température à 50°C, une solution de MEA obtenue en dissolvant 12,5 g de MEA dans 70 g de THF. On laisse ensuite réagir le tout pendant 7H, sous agitation et à 50°C.

La fin de la réaction peut être controlée en vérifiant par analyse infra-rouge l'absence de bandes d'adsorption -N=C=O à 2270 cm⁻¹. Si besoin est, on peut alors rajouter de l'éthanol au milieu réactionnel pour terminer la réaction et consommer totalement les groupes -N=C=O encore disponibles; dans ce cas, on peut par exemple rajouter de l'ordre de 10 ml d'éthanol et laisser à nouveau réagir le tout pendant 4 H à 50°C.

En fin de réaction, on obtient une solution organique (THF) du polycondensat désiré, lequel est ensuite récupéré et purifié par précipitation de ladite solution dans 5 l d'un mélange équi-volume (50/50) éther de pétrole/éther éthylique. Le rendement de récupération est de 93% en poids après séchage.

L'indice d'amine du polycondensat obtenu est de 45,7 (théorique : 43).

Son poids moléculaire moyen en nombre est de 12600.

Le polymère obtenu est soluble dans des solvants tels que le diméthoxyéthane, l'éthanol, l'isododécane et l'acétate d'éthyle.

### Exemple 4

On donne ici trois exemples de formulations capillaires.

### Lotion de mise en forme :

- - Polymère de l'exemple 1: 2 g
- - Diméthoxyéthane: 50 g
- -AMP: 0,146 g
- - Ethanol: 40,67 g
- - Isododécane: 7,18 g

Cette lotion est obtenue par dissolution du polymère dans le diméthoxyéthane puis ajout du neutralisant puis dilution de la solution par l'addition de l'éthanol et de l'isododécane.

Cette composition, appliquée sur les cheveux, après un shampooing, apporte un bon maintien de la coiffure et une très bonne brillance aux cheveux.

### Lotion de mise en forme :

- - Polymère de l'exemple 2: 2 g
- - Diméthoxyéthane: 50 g
- -AMP: 0,069 g
- - Ethanol: 43,14 g
- - Isododécane: 4,80 g

Cette lotion est obtenue par dissolution du polymère dans le diméthoxyéthane puis ajout du neutralisant puis dilution de la solution par l'addition de l'éthanol et de l'isododécane.

Cette composition, appliquée sur les cheveux, après un shampooing, apporte un bon maintien de la coiffure et une très bonne brillance aux cheveux.

### Lotion de mise en forme :

- - Polymère de l'exemple 3: 4,2 g
- - Diméthoxyéthane: 47,66 g
- - Ethanol: 42,90 g
- - Isododécane: 4,76 g
- - Solution de HCl 2 M: 0,476 g

Cette lotion est obtenue par dissolution du polymère dans le diméthoxyéthane puis dilution de la solution par l'addition de l'éthanol et de l'isododécane. On ajoute ensuite la solution de HCl pour neutraliser la solution finale.

Cette composition, appliquée sur les cheveux, après un shampooing, apporte un bon maintien de la coiffure et une très bonne brillance aux cheveux.

### Exemple 5

On donne ici quatre exemples de bases de soin des ongles.

### Base de soin des ongles

- - Polymère de l'exemple 1: 30 g
- - Acétate d'éthyle: 70 g

### Base de soin des ongles

- - Polymère de l'exemple 1: 30 g
- - Acétate d'éthyle: 66,5 g
- - Isododécane: 3,5 g

### Base de soin des ongles

- - Polymère de l'exemple 2: 30 g
- - Acétate d'éthyle: 70 g

### Base de soin des ongles

- - Polymère de l'exemple 2: 30 g
- - Acétate d'éthyle: 66,5 g
- - Isododécane: 3,5 g

## Revendications

1. Composition cosmétique ou dermatologique, caractérisée par le fait qu'elle comprend, dans un support cosmétiquement acceptable, au moins une solution ou une émulsion solvant organique-dans-eau d'au moins un polycondensat multi-séquencé dont la chaîne est constituée par la répétition d'au moins une séquence polysiloxane et d'au moins une séquence polyuréthane et/ou polyurée ; ladite séquence polyuréthane et/ou polyurée comportant en outre des groupements ionisables et ledit polycondensat étant dissous dans un système solvant aqueux, organique ou hydroorganique.

2. Composition selon la revendication 1, caractérisée en ce que le poids moléculaire moyen en nombre dudit polycondensat est compris entre 2000 et 500 000.

3. Composition selon la revendication 2, caractérisée en ce que ledit poids moléculaire est compris entre 3000 et 250 000.

4. Composition selon l'une quelconque des revendications 1 à 3, caractérisée en ce que le rapport en nombre entre les séquences de type polyuréthane et/ou polyurée et les séquences de type polysiloxane dans le polycondensat est compris entre 1 et 10.

5. Composition selon la revendication 4, caractérisée en ce que ledit rapport est compris entre 1 et 3.

6. Composition selon l'une quelconque des revendications 1 à 5, caractérisée en ce que lesdits groupements ionisables sont des fonctions carboxyliques ou des fonctions sulfoniques, sous forme libre ou bien partiellement ou totalement neutralisées.

7. Composition selon l'une quelconque des revendications 1 à 5, caractérisée en ce que lesdits groupements ionisables sont des amines tertiaires, soit non-neutralisées, soit totalement ou partiellement neutralisées, soit quaternisées.

8. Composition selon l'une des revendications 6 ou 7, caractérisée en ce que le taux de neutralisation ou de quaternisation des groupements ionisables est compris entre 0 et 100%.

9. Composition selon la revendication 8, caractérisée en ce que ledit taux est compris entre 10 et 100%.

10. Composition selon l'une quelconque des revendications 1 à 9, caractérisée en ce que la séquence polysiloxane répond à la formule générale (I) suivante : 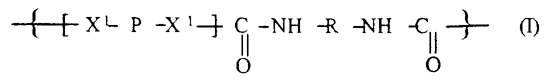 dans laquelle :
- P est un segment polysiloxanique,
- X¹ représente, séparement ou conjointement, -O- ou -NH-,
- et R est un radical divalent choisi parmi les radicaux alkylènes de type aromatique, aliphatique ou cycloaliphatique.

11. Composition selon la revendication 10, caractérisée en ce que ledit segment polysiloxanique P répond à la formule générale (I') suivante : 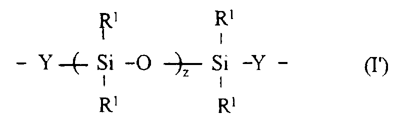 dans laquelle les radicaux R¹, qui peuvent être identiques ou différents, sont choisis parmi d'une part les radicaux hydrocarbonés monovalents en C₁-C₂₀ exempts ou substantiellement exempts d'insaturations éthyléniques et, d'autre part, les radicaux aromatiques, Y représente un radical hydrocarboné bivalent, et z un nombre entier tel que le poids moléculaire moyen du segment polysiloxane soit compris entre 300 et 10 000.

12. Composition selon la revendication 11, caractérisée en ce que ledit radical bivalent Y est choisi parmi les radicaux alkylènes de formule -(CH₂)ₐ-, dans laquelle a représente un nombre entier pouvant être compris entre 1 et 10.

13. Composition selon l'une des revendications 11 ou 12, caractérisée en ce que les radicaux R¹ sont choisis parmi les radicaux alkyles, en particulier les radicaux méthyle, éthyle, propyle, isopropyle, butyle, pentyle, hexyle, octyle, décyle, dodécyle et octadécyle, les radicaux cycloalkyles, en particulier le radical cyclohexyle, les radicaux aryles, notamment phényle et naphtyle, les radicaux arylalkyles, notamment benzyle et phényléthyle, ainsi que les radicaux tolyle et xylyle.

14. Composition selon la revendication 10 ou 11, caractérisée en ce que le segment polysiloxane P répond à la formule (I'') suivante : 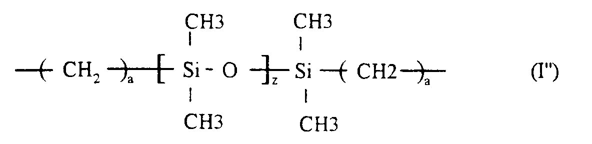 dans laquelle a et z sont des valeurs telles que définies ci-avant.

15. Composition selon l'une quelconque des revendications 1 à 14, caractérisée en ce que les séquences polyuréthanes et/ou polyurées répondent à la formule générale (II) suivante : 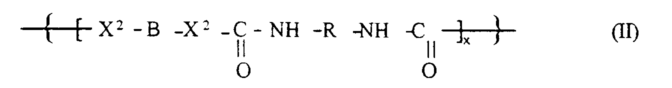 dans laquelle :
- X² représente, séparément ou conjointement, -O- ou -NH-,
- R est tel que défini ci-dessus pour les séquences de formule (I),
- x est un nombre entier compris entre 1 à 10,
- et B est un radical hydrocarboné bivalent porteur d'une charge ionique, positive ou négative.

16. Composition selon la revendication 15, caractérisée en ce que x est compris entre 1 et 3.

17. Composition selon l'une des revendications 15 ou 16, caractérisée en ce que ledit radical B est porteur de groupement présentant une ou des fonction(s) carboxylique(s) et/ou une ou des fonctions sulfoniques, lesdites fonctions carboxyliques et/ou sulfoniques étant sous forme libre ou bien neutralisées partiellement ou totalement par une base minérale ou organique.

18. Composition selon la revendication 17, caractérisée en ce que le radical B répond à la formule (III) : 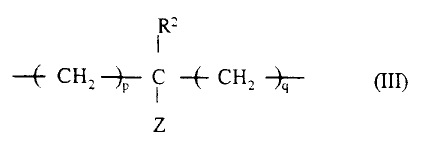 dans laquelle R² représente un radical alkyle, linéaire ou ramifié, en C₁-C₃, Z une fonction acide carboxylique (-COOH) ou acide sulfonique (-SO₃H) ou un sel desdites fonctions acides, et p et q, qui peuvent être identiques ou différents, des nombres entiers compris entre 1 et 5.

19. Composition selon la revendication 17, caractérisée en ce que le radical B répond à la formule (III') : 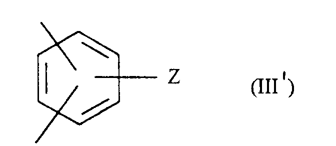 dans laquelle Z a la signification ci-dessus.

20. Composition selon l'une des revendications 15 ou 16, caractérisée en ce que ledit radical B est porteur de groupements amines tertiaires, lesdites amines tertiaires étant soit non-neutralisées, soit partiellement ou totalement neutralisées par une base minérale ou organique, soit quaternisées.

21. Composition selon la revendication 20, caractérisée en ce que le radical B répond à la formule (IV) suivante : 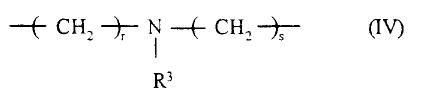 dans laquelle R³ représente un radical alkyle, linéaire ou ramifié, en C₁-C₄, et r et s deux nombres entiers, identiques ou différents, qui peuvent être compris entre 1 et 10.

22. Composition selon la revendication 21, caractérisée en ce que, sous forme neutralisée ou quaternisée, le radical B répond à la formule (IV') suivante : 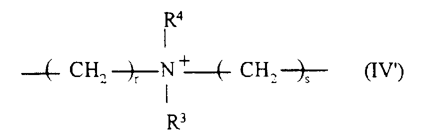 formule dans laquelle R³ a la signification ci-dessus, et R⁴ représente soit l'hydrogène (neutralisation) soit un radical alkyle, linéaire ou ramifié, en C₁-C₁₀ ou un cycle aromatique (quaternisation).

23. Composition selon l'une quelconque des revendications 10 à 22, caractérisée en ce que le radical R est choisi parmi les radicaux de formules suivantes : 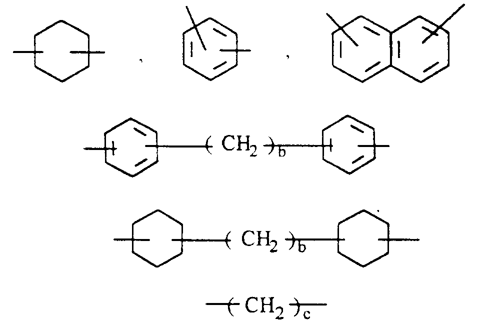 dans lesquelles b est un nombre entier compris entre 0 et 3, et c un nombre entier compris entre 1 et 20, de préférence compris entre 2 et 12.

24. Composition selon la revendication 23, caractérisée en ce que ledit radical R est choisi parmi les radicaux hexaméthylène, 4,4'-biphénylèneméthane, 2,4- et/ou 2,6-tolylène, 1,5-naphtylène, p-phénylène, méthylène 4,4-biscyclohéxyle et le radical divalent dérivé de l'isophorone.

25. Composition selon l'une quelconque des revendications 1 à 24, caractérisée en ce que ledit polycondensat a été obtenu selon un procédé consistant à faire réagir, dans une première étape, (i) un polymère polysiloxane du type α,ω-dihydroxypolysiloxane ou α,ω-diaminopolysiloxane ou α,ω-aminohydroxy- ou hydroxyaminopolysiloxane et (ii) un diisocyanate, ledit diisocyanate étant présent en quantité stoechiométrique ou en excès stoechiométrique, ce par quoi l'on obtient un polysiloxane présentant une fonction isocyanate à chacune de ses extrémités de chaîne ; puis, dans une deuxième étape, à coupler les chaînes du polysiloxane obtenu précédemment par réaction avec un agent coupleur choisi parmi les diols et/ou les diamines et/ou les alcoolamines, ledit agent coupleur étant porteur de groupements cationisables ou anioniables; et éventuellement, dans une troisième étape, à ioniser, partiellement ou totalement, les groupements cationisables ou anionisables du polycondensat obtenu à l'issue de la deuxième étape.

26. Composition selon la revendication 25, caractérisé en ce que le taux de ionisation est compris entre 0 et 100%, de préférence entre 10 et 100%.

27. Composition selon l'une des revendications 25 ou 26, caractérisée en ce que le polymère polysiloxane de départ répond à la formule :
X³-P-X³
dans laquelle P a la signification donnée ci-avant, et X³ représente, conjointement ou séparément, -OH ou -NH₂.

28. Composition selon l'une des revendications 25 à 27, caractérisée en ce que le diisocyanate répond à la formule :
O=C=N-R-N=C=O
dans laquelle R a la signification donnée ci-avant.

29. Composition selon l'une des revendications 25 à 28, caractérisée en ce que l'agent coupleur répond à la formule :
X⁴-B-X⁴
dans laquelle B a la signification donnée ci-avant, et X⁴ représente -OH ou -NH₂.

30. Composition selon l'une quelconque des revendications 1 à 29, caractérisée en ce que le système solvant du polycondensat contient un solvant organique ou un mélange de solvants organiques choisis dans le groupe constitué par l'acétone, la méthyléthylcétone, l'acétate de méthyle, l'acétate de butyle, l'acétate d'éthyle, l'isopropanol, l'éthanol, le diméthoxyéthane, le diéthoxyéthane, un ester d'éthylèneglycol ou de propylèneglycol, un ether d'éthylèneglycol ou de propylèneglycol, ou un ether ester d'éthylèneglycol ou de propylèneglycol.

31. Composition selon l'une quelconque des revendications 1 à 30, caractérisée en ce que le système solvant du polycondensat contient un solvant organique ou un mélange de solvants organiques miscibles à l'eau.

32. Composition selon l'une quelconque des revendications 1 à 30, caractérisée en ce que le système solvant du polycondensat contient un solvant organique ou un mélange de solvants organiques non-miscibles à l'eau.

33. Composition selon l'une quelconque des revendications 1 à 32, caractérisée en ce que le système solvant contient au moins un solvant (A) dit 〈〈global〉〉 du polycondensat et au moins un solvant (B) moins polaire que (A) qui va solvater plus spécifiquement les séquences polysiloxanes du polycondensat et de préférence ayant une vitesse d'évaporation plus lente que (A).

34. Composition selon la revendication 33, selon laquelle le solvant global (A) est choisi dans le groupe constitué par l'acétone, la méthyléthylcétone, l'acétone, la méthyléthylcétone, l'acétate de méthyle, l'acétate de butyle, l'acétate d'éthyle, l'isopropanol, l'éthanol, le diméthoxyéthane, le diéthoxyéthane, un ester d'éthylèneglycol ou de propylèneglycol, un éther d'éthylèneglycol ou de propylèneglycol, un éther ester d'éthylèneglycol ou de propylèneglycol et leurs mélanges.

35. Composition selon la revendication 33 ou 34, selon laquelle le solvant (B) est choisi parmi les hydrocarbures et les silicones cycliques.

36. Composition selon l'une quelconque des revendications 1 à 35, caractérisée en ce qu'il s'agit d'une composition capillaire.

37. Composition selon l'une quelconque des revendications 1 à 35, caractérisée en ce qu'il s'agit d'une composition de maquillage.

38. Composition selon l'une quelconque des revendications 1 à 35, caractérisée en ce qu'il s'agit d'un vernis à ongle ou d'une base de soin des ongles.

39. Composition selon l'une quelconque des revendications 1 à 35, caractérisée en ce qu'il s'agit d'un mascara.

40. Composition selon l'une quelconque des revendications 1 à 35, caractérisée en ce qu'il s'agit d'une composition pour le soin de la peau.

41. Composition selon l'une quelconque des revendications 1 à 35, caractérisée en ce qu'il s'agit d'une composition anti-solaire.

42. Utilisation d'une solution ou d'émulsion de polycondensat telle que définie à l'une quelconque des revendications 1 à 35 comme agent filmogène, ou comme additif d'agent filmogène, dans une composition cosmétique.

43. Procédé de traitement des matières kératiniques, caractérisé en ce qu'il consiste à appliquer sur ces dernières une composition telle que définie à l'une quelconque des revendications 1 à 41.

## Claims

1. Cosmetic or dermatological composition, characterized in that it comprises, in a cosmetically acceptable support, at least one solution or one organic solvent-in-water emulsion of at least one multiblock polycondensate whose chain consists of the repetition of at least one polysiloxane block and of at least one polyurethane and/or polyurea block; the said polyurethane and/or polyurea block also including ionizable groups and the said polycondensate being dissolved in an aqueous, organic or aqueous-organic solvent system.

2. Composition according to Claim 1, characterized in that the number-average molecular weight of the said polycondensate is between 2000 and 500,000.

3. Composition according to Claim 2, characterized in that the said molecular weight is between 3000 and 250,000.

4. Composition according to any one of Claims 1 to 3, characterized in that the numerical ratio between the polyurethane and/or polyurea type blocks and the polysiloxane type blocks in the polycondensate is between 1 and 10.

5. Composition according to Claim 4, characterized in that the said ratio is between 1 and 3.

6. Composition according to any one of Claims 1 to 5, characterized in that the said ionizable groups are carboxylic functions or sulphonic functions, in free or partially or totally neutralized form.

7. Composition according to any one of Claims 1 to 5, characterized in that the said ionizable groups are tertiary amines which are either non-neutralized or totally or partially neutralized or quaternized.

8. Composition according to either of Claims 6 and 7, characterized in that the degree of neutralization or of quaternization of the ionizable groups is between 0 and 100%.

9. Composition according to Claim 8, characterized in that the said degree is between 10 and 100%.

10. Composition according to any one of Claims 1 to 9, characterized in that the polysiloxane block corresponds to the general formula (I) below: 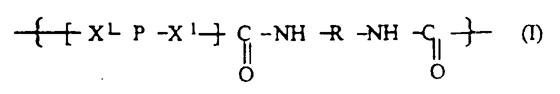 in which:
- P is a polysiloxane segment,
- X¹ represents, separately or in combination, -O- or
- and R is a divalent radical chosen from alkylene radicals of aromatic, aliphatic or cycloaliphatic type.

11. Composition according to Claim 10, characterized in that the said polysiloxane segment P corresponds to the general formula (I') below: 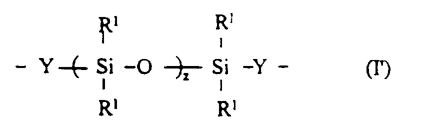 in which the radicals R¹, which may be identical or different, are chosen, on the one hand, from monovalent C₁-C₂₀ hydrocarbon radicals which are free or substantially free of ethylenic unsaturations, and, on the other hand, aromatic radicals, Y represents a divalent hydrocarbon radical and z is an integer such that the average molecular weight of the polysiloxane segment is between 300 and 10,000.

12. Composition according to Claim 11, characterized in that the said divalent radical Y is chosen from alkylene radicals of formula -(CH₂)ₐ-, in which a represents an integer which may be between 1 and 10.

13. Composition according to either of Claims 11 and 12, characterized in that the radicals R¹ are chosen from alkyl radicals, in particular methyl, ethyl, propyl, isopropyl, butyl, pentyl, hexyl, octyl, decyl, dodecyl and octadecyl radicals, cycloalkyl radicals, in particular the cyclohexyl radical, aryl radicals, in particular phenyl and naphthyl, arylalkyl radicals, in particular benzyl and phenylethyl, and tolyl and xylyl radicals.

14. Composition according to Claim 10 or 11, characterized in that the polysiloxane segment P corresponds to formula (I'') below: 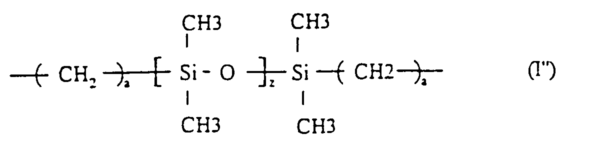 in which a and z are values as defined above.

15. Composition according to any one of Claims 1 to 14, characterized in that the polyurethane and/or polyurea blocks correspond to the general formula (II) below: 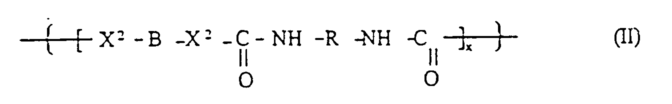 in which:
- X² represents, separately or in combination, -O- or
- R is as defined above for the blocks of formula (I),
- x is an integer between 1 and 10,
- and B is a divalent hydrocarbon radical bearing a positive or negative ionic charge.

16. Composition according to Claim 15, characterized in that x is between 1 and 3.

17. Composition according to either of Claims 15 and 16, characterized in that the said radical B bears a group having one or more carboxylic function(s) and/or one or more sulphonic functions, the said carboxylic and/or sulphonic functions being in free form or alternatively partially or totally neutralized with an inorganic or organic base.

18. Composition according to Claim 17, characterized in that the radical B corresponds to formula (III): 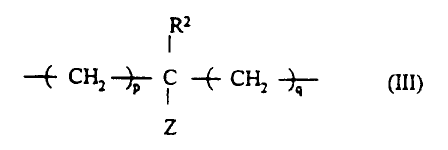 in which R² represents a linear or branched C₁-C₃ alkyl radical, Z represents a carboxylic acid (-COOH) function or sulphonic acid (-SO₃H) function or a salt of the said acidic functions, and p and q, which may be identical or different, are integers between 1 and 5.

19. Composition according to Claim 17, characterized in that the radical B corresponds to formula (III'): 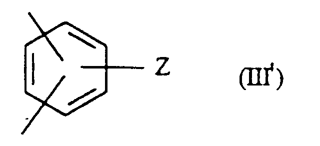 in which Z has the above meaning.

20. Composition according to either of Claims 15 and 16, characterized in that the said radical B bears tertiary amine groups, the said tertiary amines being either non-neutralized or partially or totally neutralized with an inorganic or organic base, or quaternized.

21. Composition according to Claim 20, characterized in that the radical B corresponds to formula (IV) below: 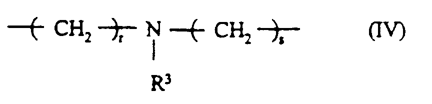 in which R³ represents a linear or branched C₁-C₄ alkyl radical and r and s are both integers, which may be identical or different, and which may be between 1 and 10.

22. Composition according to Claim 21, characterized in that, in neutralized or quaternized form, the radical B corresponds to formula (IV') below: 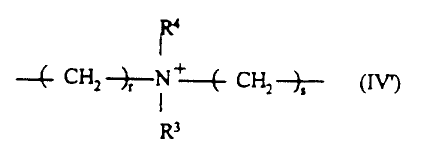 in which formula R³ has the above meaning, and R⁴ represents either hydrogen (neutralization) or a linear or branched C₁-C₁₀ alkyl radical or an aromatic ring (quaternization).

23. Composition according to any one of Claims 10 to 22, characterized in that the radical R is chosen from the radicals of the following formulae: 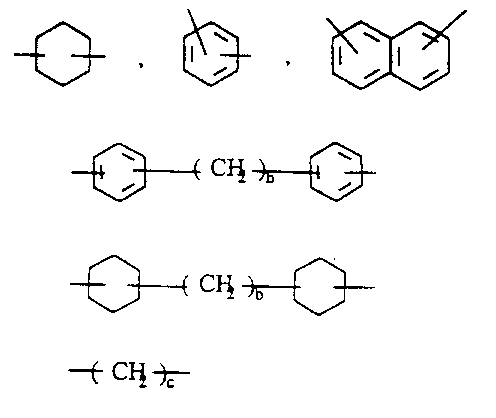 in which b is an integer between 0 and 3, and c is an integer between 1 and 20, preferably between 2 and 12.

24. Composition according to Claim 23, characterized in that the said radical R is chosen from hexamethylene, 4,4'-biphenylenemethane, 2,4- and/or 2,6-tolylene, 1,5-naphthylene, p-phenylene and methylene-4,4-bis-cyclohexyl radicals and the divalent radical derived from isophorone.

25. Composition according to any one of Claims 1 to 24, characterized in that the said polycondensate is obtained according to a process consisting in reacting, in a first step, (i) a polysiloxane polymer of the α,ω-dihydroxypolysiloxane or α,ω-diaminopolysiloxane or α,ω-aminohydroxy- or hydroxyamino-polysiloxane type, and (ii) a diisocyanate, the said diisocyanate being present in stoichiometric amount or in stoichiometric excess, by which reaction a polysiloxane is obtained having an isocyanate function at each of its chain ends; then, in a second step, in coupling the chains of the polysiloxane obtained above by reaction with a coupler chosen from diols and/or diamines and/or aminoalcohols, the said coupler bearing cationizable or anionizable groups; and optionally, in a third step, in partially or totally ionizing the cationizable or anionizable groups of the polycondensate obtained after the second step.

26. Composition according to Claim 25, characterized in that the degree of ionization is between 0 and 100%, preferably between 10 and 100%.

27. Composition according to either of Claims 25 and 26, characterized in that the starting polysiloxane polymer corresponds to the formula:
X³-P-X³
in which P has the meaning given above, and X³ represents, in combination or separately,
-OH or -NH₂.

28. Composition according to one of Claims 25 to 27, characterized in that the diisocyanate corresponds to the formula:
O=C=N-R-N=C=O
in which R has the meaning given above.

29. Composition according to one of Claims 25 to 28, characterized in that the coupler corresponds to the formula:
X⁴-B-X⁴
in which B has the meaning given above and X⁴ represents -OH or -NH₂.

30. Composition according to any one of Claims 1 to 29, characterized in that solvent system for the polycondensate contains an organic solvent or a mixture of organic solvents chosen from the group consisting of acetone, methyl ethyl ketone, methyl acetate, butyl acetate, ethyl acetate, isopropanol, ethanol, dimethoxyethane, diethoxyethane, an ethylene glycol or a propylene glycol ester, an ethylene glycol or a propylene glycol ether, or an ethylene glycol or a propylene glycol ester ether.

31. Composition according to any one of Claims 1 to 30, characterized in that the solvent system for the polycondensate contains an organic solvent or a mixture of organic solvents which are miscible with water.

32. Composition according to any one of Claims 1 to 30, characterized in that the solvent system for the polycondensate contains an organic solvent or a mixture of organic solvents which are immiscible in water.

33. Composition according to any one of Claims 1 to 32, characterized in that the solvent system contains at least one solvent (A) known as a global solvent for the polycondensate and at least one solvent (B) which is less polar than (A) and which will more specifically dissolve the polysiloxane blocks of the polycondensate and preferably has a slower rate of evaporation than (A).

34. Composition according to Claim 33, according to which the global solvent (A) is chosen from the group consisting of acetone, methyl ethyl ketone, methyl acetate, butyl acetate, ethyl acetate, isopropanol, ethanol, dimethoxyethane, diethoxyethane, an ethylene glycol or a propylene glycol ester, an ethylene glycol or a propylene glycol ether, or an ethylene glycol or a propylene glycol ester ether, and mixtures thereof.

35. Composition according to Claim 33 or 34, according to which the solvent (B) is chosen from cyclic silicones and hydrocarbons.

36. Composition according to any one of Claims 1 to 35, characterized in that it is a hair composition.

37. Composition according to any one of Claims 1 to 35, characterized in that it is a make-up composition.

38. Composition according to any one of Claims 1 to 35, characterized in that it is a nail varnish or a nail care base.

39. Composition according to any one of Claims 1 to 35, characterized in that it is a mascara.

40. Composition according to any one of Claims 1 to 35, characterized in that it is a skincare composition.

41. Composition according to any one of Claims 1 to 35, characterized in that it is an antisun composition.

42. Use of a polycondensate solution or emulsion as defined in any one of Claims 1 to 35, as a film-forming agent, or as an additive for a film-forming agent, in a cosmetic composition.

43. Process for treating keratin substances, characterized in that it consists in applying a composition as defined in any one of Claims 1 to 41 to these substances.

## Patentansprüche

1. Kosmetische oder dermatologische Zusammensetzungen, dadurch gekennzeichnet, daß sie in einem kosmetisch akzeptablen Träger mindestens eine organisches-Lösungsmittel-in-Wasser-Lösung oder eine organisches-Lösungsmittel-in-Wasser-Emulsion mindestens eines Mehrblock-Polykondensats enthalten, dessen Kette aus wiederkehrenden Einheiten mindestens eines Polysiloxanblocks und mindestens eines Polyurethan- und/oder Polyharnstoffblocks besteht, wobei der Polyurethan- und/oder Polyharnstoffblock ferner ionisierbare Gruppen aufweist und wobei das Polykondensat in einem wässerigen, organischen oder wässerig-organischen Lösungsmittelsystem gelöst ist.

2. Zusammensetzungen nach Anspruch 1, dadurch gekennzeichnet, daß das Zahlenmittel des Molekulargewichts des Polykondensats im Bereich von 2 000 bis 500 000 liegt.

3. Zusammensetzungen nach Anspruch 2, dadurch gekennzeichnet, daß dieses Zahlenmittel des Molekulargewlchts im Bereich von 3 000 bis 250 000 liegt.

4. Zusammensetzungen nach einem der Ansprüche 1 bis 3, dadurch gekennzeichnet, daß das Verhältnis der Anzahl der Blöcke vom Polyurethan- und/oder Polyharnstofftyp zu den Blöcken vom Polysiloxantyp in dem Polykondensat im Bereich von 1 bis 10 liegt.

5. Zusammensetzungen nach Anspruch 4, dadurch gekennzeichnet, daß dieses Verhältnis im Bereich von 1 bis 3 liegt.

6. Zusammensetzungen nach einem der Ansprüche 1 bis 5, dadurch gekennzeichnet, daß es sich bei den ionisierbaren Gruppen um Carboxy-Gruppen oder Sulfonsäure-Gruppen handelt, die in freier Form oder auch zum Teil oder vollständig neutralisiert vorliegen.

7. Zusammensetzungen nach einem der Ansprüche 1 bis 5, dadurch gekennzeichnet, daß es sich bei diesen ionisierbaren Gruppen entweder um nicht neutralisierte, vollständig oder zum Teil neutralisierte oder quaternisierte tertiäre Amine handelt.

8. Zusammensetzungen nach einem der Ansprüche 6 oder 7, dadurch gekennzeichnet, daß der Grad der Neutralisierung oder der Quaternisierung der ionisierbaren Gruppen im Bereich von 0 bis 100 % liegt.

9. Zusammensetzungen nach Anspruch 8, dadurch gekennzeichnet, daß dieser Grad im Bereich von 10 bis 100 % liegt.

10. Zusammensetzungen nach einem der Ansprüche 1 bis 9, dadurch gekennzeichnet, daß der Polysiloxanblock der folgenden allgemeinen Formel (I) entspricht: 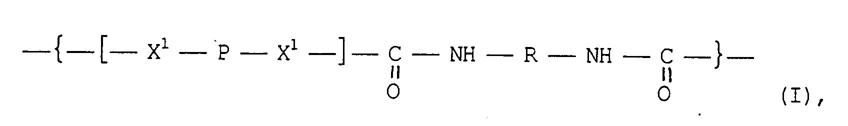 worin bedeuten:
- P ein Polysiloxansegment,
- die Gruppen X¹, unabhängig voneinander oder gleichzeitig, -O- oder -NH-, und
- R eine zweiwertige Gruppe, die unter Alkylengruppen vom aromatischen, aliphatischen oder cycloaliphatischen Typ ausgewählt ist.

11. Zusammensetzungen nach Anspruch 10, dadurch gekennzeichnet, daß das Polysiloxansegment P der folgenden allgemeinen Formel (I') entspricht: 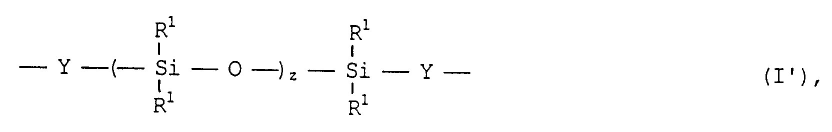 worin:
- die Gruppen R¹, die identisch oder voneinander verschieden sein können, unter einerseits einwertigen Kohlenwasserstoffgruppen mit 1 bis 20 Kohlenstoffatomen, die keine oder im wesentlichen keine ethylenischen Doppelbindungen enthalten, und andererseits aromatischen Gruppen ausgewählt sind,
- Y eine zweiwertige Kohlenwasserstoffgruppe bedeutet und
- z eine ganze Zahl bedeutet, die so ausgewählt ist, daß das mittlere Molekulargewicht des Polysiloxansegments im Bereich von 300 bis 10 000 liegt.

12. Zusammensetzungen nach Anspruch 11, dadurch gekennzeichnet, daß die zweiwertige Gruppe Y unter den Alkylengruppen der Formel -(CH₂)ₐ- ausgewählt ist, worin a eine ganze Zahl bedeutet, die im Bereich von 1 bis 10 liegen kann.

13. Zusammensetzungen nach Anspruch 11 oder 12, dadurch gekennzeichnet, daß die Gruppen R¹ ausgewählt sind unter Alkylgruppen, insbesondere Methyl, Ethyl, Propyl, Isopropyl, Butyl, Pentyl, Hexyl, Octyl, Decyl, Dodecyl und Octadecyl, Cycloalkylgruppen, insbesondere Cyclohexyl, Arylgruppen, insbesondere Phenyl und Naphthyl, Arylalkylgruppen, insbesondere Benzyl und Phenylethyl, sowie den Gruppen Tolyl und Xylyl.

14. Zusammensetzungen nach Anspruch 10 oder 11, dadurch gekennzeichnet, daß das Polysiloxansegment P der folgenden Formel (I'') entspricht: 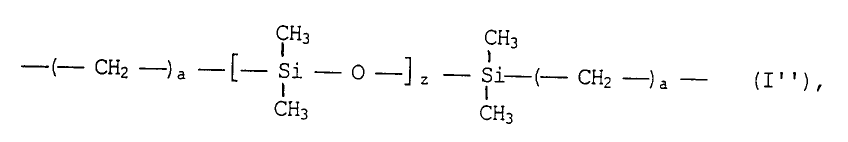 worin a und z die oben definierten Werte bedeuten.

15. Zusammensetzungen nach einem der Ansprüche 1 bis 14, dadurch gekennzeichnet, daß die Polyurethan- und/oder Polyharnstoffblöcke der folgenden allgemeinen Formel (II) entsprechen: 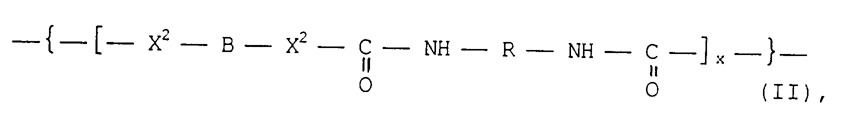 worin:
- die Gruppen X², unabhängig voneinander oder gleichzeitig, -O- oder -NH- bedeuten,
- R die oben für die Blöcke der Formel (I) angegebene Bedeutung aufweist,
- x eine ganze Zahl im Bereich von 1 bis 10 bedeutet und
- B eine zweiwertige Kohlenwasserstoffgruppe bedeutet, die eine positive oder negative Ladung aufweist.

16. Zusammensetzungen nach Anspruch 15, dadurch gekennzeichnet, daß x im Bereich von 1 bis 3 liegt.

17. Zusammensetzungen nach einem der Ansprüche 15 oder 16, dadurch gekennzeichnet, daß die Gruppe B eine Gruppe enthält, die eine oder mehrere Carboxy-Gruppen und/oder eine oder mehrere Sulfonsäure-Gruppen aufweist, wobei die Carboxy-Gruppen und/oder Sulfonsäure-Gruppen in freier Form oder auch zum Teil oder vollständig mit einer anorganischen oder organischen Base neutralisiert vorliegen.

18. Zusammensetzungen nach Anspruch 17, dadurch gekennzeichnet, daß die Gruppe B der Formel (III) entspricht: 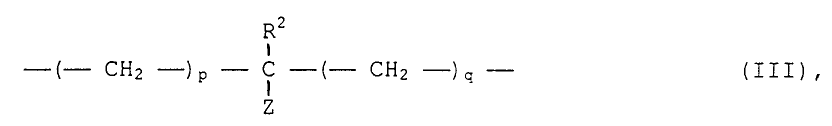 worin bedeuten:
- R² eine geradkettige oder verzweigte Alkylgruppe mit 1 bis 3 Kohlenstoffatomen,
- Z eine Carbonsäuregruppe (-COOH) oder eine Sulfonsäuregruppe (-SO₃H) oder ein Salz dieser Säuregruppen und
- p und q, die identisch oder voneinander verschieden sein können, ganze Zahlen im Bereich von 1 bis 5.

19. Zusammensetzungen nach Anspruch 17, dadurch gekennzeichnet, daß die Gruppe B der Formel (III') entspricht: 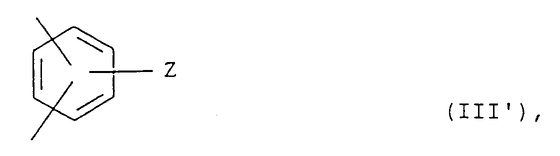 worin Z die oben angegebene Bedeutung aufweist.

20. Zusammensetzungen nach einem der Ansprüche 15 oder 16, dadurch gekennzeichnet, daß die Gruppe B tertiäre Aminogruppen enthält, wobei die tertiären Amine entweder nicht neutralisiert, zum Teil oder vollständig mit einer anorganischen oder organischen Base neutralisiert oder auch quaternisiert vorliegen.

21. Zusammensetzungen nach Anspruch 20, dadurch gekennzeichnet, daß die Gruppe B der folgenden Formel (IV) entspricht: 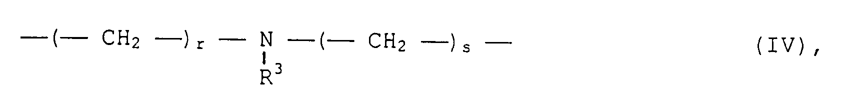 worin bedeuten:
- R³ eine geradkettige oder verzweigte Alkylgruppe mit 1 bis 4 Kohlenstoffatomen und
- r und s zwei ganze Zahlen, die identisch oder voneinander verschieden sind und die im Bereich von 1 bis 10 liegen können.

22. Zusammensetzungen nach Anspruch 21, dadurch gekennzeichnet, daß die Gruppe B in neutralisierter oder quaternisierter Form der folgenden Formel (IV') entspricht: 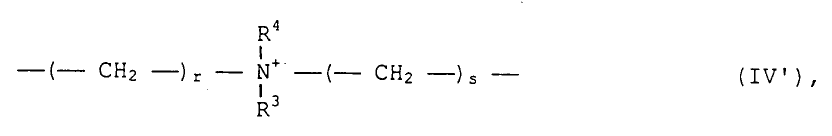 worin:
- R³ die oben angegebene Bedeutung aufweist und
- R⁴ entweder Wasserstoff (Neutralisierung) oder aber eine geradkettige oder verzweigte Alkylgruppe mit 1 bis 10 Kohlenstoffatomen oder einen aromatischen Ring (Quaternisierung) bedeutet.

23. Zusammensetzungen nach einem der Ansprüche 10 bis 22, dadurch gekennzeichnet, daß die Gruppe R unter den Gruppen der folgenden Formeln ausgewählt ist: 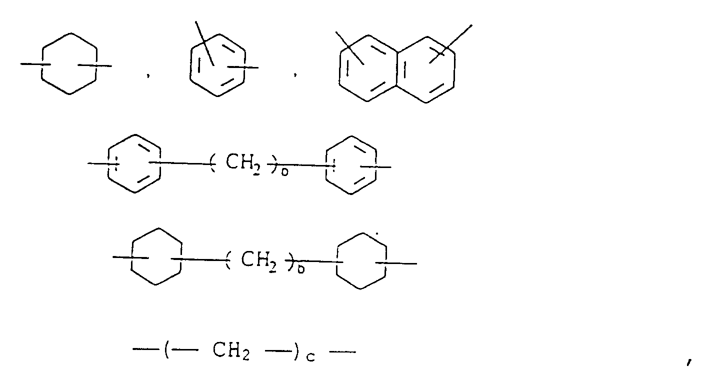 worin bedeuten:
- b Null oder eine ganze Zahl von 1 bis 3 und
- c eine ganze Zahl im Bereich von 1 bis 20, vorzugsweise im Bereich von 2 bis 12.

24. Zusammensetzungen nach Anspruch 23, dadurch gekennzeichnet, daß die Gruppe R ausgewählt ist unter Hexamethylen, 4,4'-Biphenylenmethan, 2,4-Tolylen und/oder 2,6-Tolylen, 1,5-Naphthylen, p-Phenylen, 4,4-Bis-cyclohexylmethylen und der zweiwertigen, von Isophoron abgeleiteten Gruppe.

25. Zusammensetzungen nach einem der Ansprüche 1 bis 24, dadurch gekennzeichnet, daß das Polykondensat nach einem Verfahren hergestellt wurde, das darin besteht, in einem ersten Schritt (i) ein Polysiloxanpolymer vom Typ α,ω-Dihydroxypolysiloxan, α,ω-Diaminopolysiloxan, α,ω-Aminohydroxypolysiloxan oder α,ω-Hydroxyaminopolysiloxan) mit (ii) einem Diisocyanat umzusetzen, wobei das Diisocyanat in einer stöchiometrischen Menge oder in einem stöchiometrischen Überschuß vorliegt, wodurch ein Polysiloxan erhalten wird, das an jedem Kettenende eine Isocyanat-Gruppe aufweist, und dann in einem zweiten Schritt die Ketten des zuvor erhaltenen Polysiloxans durch Umsetzung mit einem Kuppler, der unter Diolen und/oder Diaminen und/oder Alkoholaminen ausgewählt ist, zu verbinden, wobei der Kuppler in Kationen umwandelbare Gruppen oder in Anionen umwandelbare Gruppen aufweist, und gegebenenfalls in einem dritten Schritt die in Kationen umwandelbaren Gruppen oder in Anionen umwandelbaren Gruppen des nach dem zweiten Schritt erhaltenen Polykondensats zum Teil oder vollständig zu ionisieren.

26. Zusammensetzungen nach Anspruch 25, dadurch gekennzeichnet, daß der Grad der Ionisierung im Bereich von 0 bis 100 %, vorzugsweise im Bereich von 10 bis 100 %, liegt.

27. Zusammensetzungen nach einem der Ansprüche 25 oder 26, dadurch gekennzeichnet, daß das am Anfang vorliegende Polysiloxan-Polymer der Formel:
X³ ― P ― X³
entspricht, worin P die oben angegebene Bedeutung aufweist und die Gruppen X³, unabhängig voneinander oder gleichzeitig, -OH oder -NH₂ bedeuten.

28. Zusammensetzungen nach einem der Ansprüche 25 bis 27, dadurch gekennzeichnet, daß das Diisocyanat der Formel:
O=C=N-R-N=C=O
entspricht, worin R die oben angegebene Bedeutung aufweist.

29. Zusammensetzungen nach einem der Ansprüche 25 bis 28, dadurch gekennzeichnet, daß der Kuppler der Formel:
X⁴ ― B ― X⁴
entspricht, worin B die oben angegebene Bedeutung aufweist und X⁴ -OH oder -NH₂ bedeutet.

30. Zusammensetzungen nach einem der Ansprüche 1 bis 29, dadurch gekennzeichnet, daß das Lösungsmittelsystem für das Polykondensat ein organisches Lösungsmittel oder ein Gemisch von organischen Lösungsmitteln enthält, die ausgewählt sind unter Aceton, Methylethylketon, Methylacetat, Butylacetat, Ethylacetat, Isopropanol, Ethanol, Dimethoxyethan, Diethoxyethan, einem Ethylenglykolester oder Propylenglykolester, einem Ethylenglykolether oder Propylenglykolether oder einem Ethylenglykoletherester oder Propylenglykoletherester.

31. Zusammensetzungen nach einem der Ansprüche 1 bis 30, dadurch gekennzeichnet, daß das Lösungsmittelsystem für das Polykondensat ein organisches Lösungsmittel oder ein Gemisch von organischen Lösungsmitteln enthält, die mit Wasser mischbar sind.

32. Zusammensetzungen nach einem der Ansprüche 1 bis 30, dadurch gekennzeichnet, daß das Lösungsmittelsystem für das Polykondensat ein organisches Lösungsmittel oder ein Gemisch von organischen Lösungsmitteln enthält, die mit Wasser nicht mischbar sind.

33. Zusammensetzungen nach einem der Ansprüche 1 bis 32, dadurch gekennzeichnet, daß das Lösungsmittelsystem mindestens ein sog. 'globales' Lösungsmittel (A) für das Polykondensat und mindestens ein Lösungsmittel (B) enthält, das weniger polar ist als (A) und das spezieller die Polysiloxanblöcke des Polykondensats solvatisiert und vorzugsweise langsamer verdampft als (A).

34. Zusammensetzungen nach Anspruch 33, wobei das sog. 'globale' Lösungsmittel (A) ausgewählt ist unter Aceton, Methylethylketon, Methylacetat, Butylacetat, Ethylacetat, Isopropanol, Ethanol, Dimethoxyethan, Diethoxyethan, einem Ethylenglykolester oder Propylenglykolester, einem Ethylenglykolether oder Propylenglykolether, einem Ethylenglykoletherester oder Propylenglykoletherester und den Gemischen dieser Verbindungen.

35. Zusammensetzungen nach Anspruch 33 oder 34, wobei das Lösungsmittel (B) unter Kohlenwasserstoffen und cyclischen Siliconen ausgewählt ist.

36. Zusammensetzungen nach einem der Ansprüche 1 bis 35, dadurch gekennzeichnet, daß es sich um Zusammensetzungen zur Haarbehandlung handelt.

37. Zusammensetzungen nach einem der Ansprüche 1 bis 35, dadurch gekennzeichnet, daß es sich um Zusammensetzungen zum Schminken handelt.

38. Zusammensetzungen nach einem der Ansprüche 1 bis 35, dadurch gekennzeichnet, daß es sich um Nagellacke oder Pflegegrundmassen für die Nägel handelt.

39. Zusammensetzungen nach einem der Ansprüche 1 bis 35, dadurch gekennzeichnet, daß es sich um Mascaras handelt.

40. Zusammensetzungen nach einem der Ansprüche 1 bis 35, dadurch gekennzeichnet, daß es sich um Zusammensetzungen zur Pflege der Haut handelt.

41. Zusammensetzungen nach einem der Ansprüche 1 bis 35, dadurch gekennzeichnet, daß es sich um Zusammensetzungen zum Sonnenschutz handelt.

42. Verwendung einer in einem der Ansprüche 1 bis 35 definierten Lösung oder Emulsion des Polykondensats als Filmbildner oder als filmbildender Zusatz in kosmetischen Zusammensetzungen.

43. Verfahren zur Behandlung von Keratinsubstanzen, dadurch gekennzeichnet, daß es darin besteht, auf die Keratinsubstanzen eine in einem der Ansprüche 1 bis 41 definierte Zusammensetzung aufzutragen.
